(19) Europäisches Patentamt

European Patent Office (11) Publication number: **0 069 247**
Office européen des brevets **B1**

(12) **EUROPEAN PATENT SPECIFICATION**

(45) Date of publication of patent specification: **09.10.85**　(51) Int. Cl.⁴: **A 61 M 1/14,** A 61 J 1/00, G 01 N 33/50

(21) Application number: **82105176.0**

(22) Date of filing: **14.06.82**

(54) **An apparatus for measuring the concentration of a low-molecular compound in a complex medium, especially whole blood.**

(30) Priority: **03.07.81 SE 8104146**

(43) Date of publication of application:
**12.01.83 Bulletin 83/02**

(45) Publication of the grant of the patent:
**09.10.85 Bulletin 85/41**

(84) Designated Contracting States:
**AT BE CH DE FR GB IT LI LU NL**

(56) References cited:
**DE-A-2 325 035**
**DE-A-2 908 117**
**FR-A-2 082 978**
**FR-A-2 400 909**
**US-A-3 187 750**
**US-A-3 257 072**

(73) Proprietor: **Gambro Lundia AB**
**Box 10101**
**S-220 10 Lund (SE)**

(72) Inventor: **Claren, Jan Seivert**
**Magistratsvägen 11 A**
**S-222 43 Lund (SE)**
Inventor: **Olsson, Lars-Göran**
**Docentgatan 6**
**S-223 63 Lund (SE)**
Inventor: **Paulsson, Bengt Göran**
**Kämpagränden 11 C**
**S-223 76 Lund (SE)**

(74) Representative: **Boberg, Nils Gunnar Erik et al**
**Gambro AB Patent Department Box 10101**
**S-220 10 Lund (SE)**

Courier Press, Leamington Spa, England.

**Description**

Technical Field

The present invention in general relates to an apparatus for measuring the concentration of a low-molecular compound in a complex medium. The apparatus comprises a dialyzer for the dialysis of the complex medium so as to form a dialyzate on which the measurement is to be carried out, a measuring device, and a common container for the feed and removal, respectively, of a dialyzing liquid and at least one further liquid useful for the measurement, whereby said container is divided into separate chambers for said liquids.

The invention relates in particular to an apparatus of the type specified for measuring the concentration of a low-molecular compound in whole blood, especially in connection with a medical treatment. An example of such a compound may be glucose.

Background Art

An apparatus for measuring the concentration of a low-molecular compound in a complex medium is known, for example from US patent 4 229 542. The means for the feed and removal of said liquids in this known apparatus comprises for every one of the liquids used a separate container provided with suitable connections to be connected to corresponding junctions on the apparatus.

Similar apparatuses are described also in Swedish patent 402 980 as well as in US patents 4 123 353, 4 240 291 and 4 266 021. These known apparatuses too have in common that the said means for the feed and removal respectively of the liquids used comprises a separate container for every one of these liquids.

An improved apparatus according to DE—A—2 325 035 comprises a common container for the feed and removal, respectively, of dialyzing liquid and at least one further liquid useful for the measurement, whereby said container is divided into separate chambers for said liquids. The advantage obtained by using a common container instead of separate or individual containers is that the apparatus becomes easier to install and at the same time the checking and monitoring of the different liquid volumes are made simpler, since the liquids can now be kept more closely together than previously.

A disadvantage of the known apparatus according to said DE—A—2 325 035 is however that the apparatus is unnecessarily big, i.e. space-requiring, and an object of this invention is therefore to provide an apparatus which on the one hand avoids the use of separate or individual containers (as disclosed in said US patents 4 229 542, 4 123 353, 4 240 291 ns 4 266 021) and Swedish patent 402 980) and which on the other hand can be made smaller, i.e less space-requiring, than the apparatus according to said DE—A—2 325 035.

Disclosure of Invention

In accordance with the present invention is thus provided an apparatus for measuring the concentration of a low-molecular compound in a complex medium, especially whole blood, this apparatus comprising a dialyzer for the dialysis of the complex medium so as to form a dialyzate on which the measurement is to be carried out, a measuring device for carrying out said measurement, and a common container for the feed and removal, respectively, of dialyzing liquid and at least one further liquid useful for the measurement, wherein said container is divided into separate chambers for said liquids. The apparatus is characterized in that said container is folded along welded zones to a substantially bellows-like packet with the chambers arranged next to one another.

The bag is made preferably of a transparent material, which has the effect that the monitoring and checking of the liquid volumes enclosed in the different chambers of the bag readily take place by visual means.

In accordance with a specially preferred embodiment, holes are provided in the welded zones in such a manner that these holes form two through-going holes which are intended for the suspension of the container in a manner which will be described in more detail.

Brief Description of Drawings

In the following the invention will be described further with reference to the enclosed drawings, wherein

Fig. 1 is a block diagram of the apparatus in accordance with a first preferred embodiment,

Fig. 2 is a block diagram of the apparatus in accordance with a second preferred embodiment, and

Fig. 3 shows schematically a preferred embodiment of the common container forming part as a component of the apparatus in accordance with Fig. 1 and Fig. 2.

Best Mode of Carrying Out the Invention

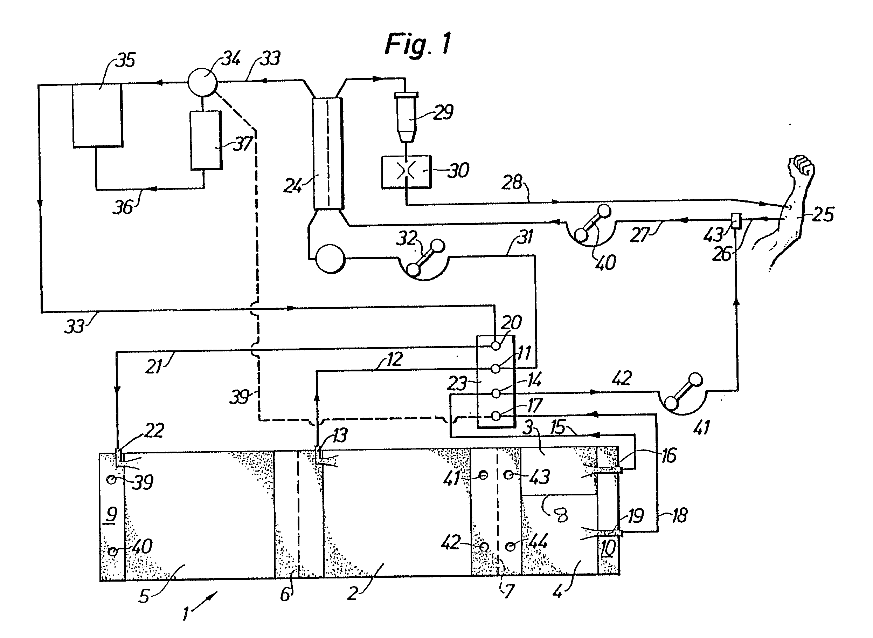

The apparatus in accordance with the invention shown in Fig. 1 is intended chiefly to be used in connection with whole blood for measuring the concentration of glucose and will be described therefore with special reference to this application.

In the example shown the apparatus comprises a common container 1 for the liquids which are to be used. The container is in the shape of a rectangular elongated bag with the interior of the bag divided preferably into four separate chambers 2, 3, 4, 5 by means of suitable welding, as indicated at 6, 7, 8. Furthermore, the bag is made of a flexible plastic material in the form of an extruded flexible tube with the ends closed by welding, as indicated at 9, 10.

The first chamber 2 in this example is adapted to contain dialyzing solution, e.g. NaCl, and is connected to a corresponding junction 11 on the apparatus via a conduit 12 and connecting nipple 13. The second chamber 3 is adapted to contain for example heparin or a similar anticoagulant liquid and is connected to a junction 14 on the apparatus via a conduit 15 and connecting nipple 16. The third chamber 4 is adapted to contain a calibrating solution for the measurement and is connected to a corresponding junction 17 on the apparatus via a conduit 18 and connecting nipple 19. The fourth chamber 5 is adapted to collect spent liquid from the chambers 2, 3, 4 and is connected to a corresponding junction 20 on the apparatus via a conduit 21 and a connecting nipple 22.

The junctions 11, 14, 17, 20 are preferably arranged in a common junction block 23, as shown.

The apparatus moreover comprises in a known manner a dialyzer 24 with an inlet side for the whole blood connected to a source 25 for the whole blood, for example a patient, via a conduit 26, 27. The outlet side for the whole blood is connected to the source 25 via a return conduit 28 comprising an air-trap 29 and clip 30. The inlet side for dialyzing solution of the dialyzer 24 is connected to the junction 11 via a conduit 31 comprising a pump 32. The outlet side for dialyzing solution is connected in a corresponding manner to the junction 20 via a conduit 33 comprising a valve 34 and a measuring apparatus 35, for example an electrode. As can be seen, the conduit 33 comprises a shunt circuit 36 between the valve 34 and the measuring apparatus 35 and an enzyme bed 37. Since these components in themselves do not constitute any part of the invention, but are well known in the field both with regard to design and operation, they do not require description here.

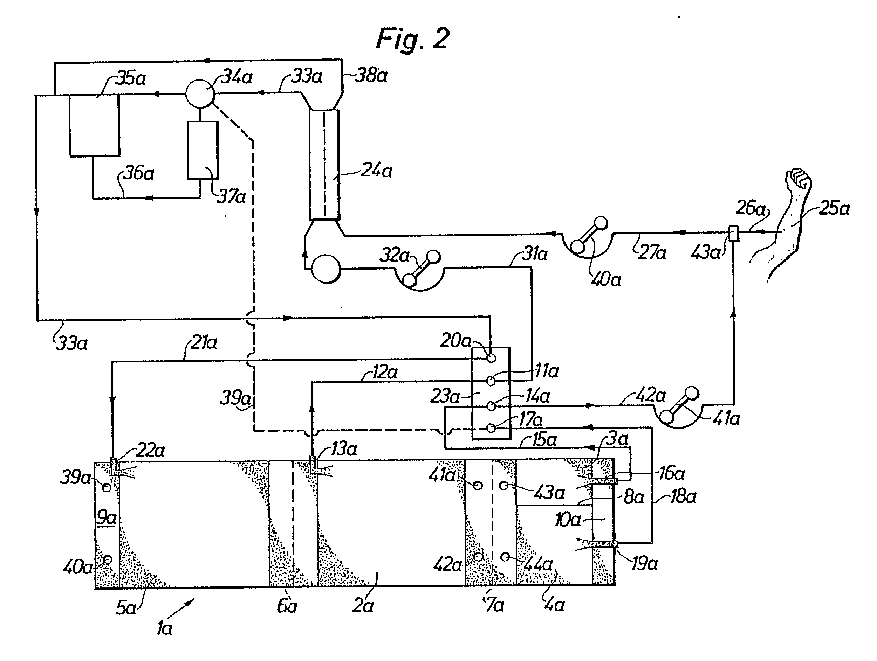

The apparatus in accordance with the invention shown in Fig. 2 is built up essentially in the same manner as that described above in accordance with Fig. 1. The same reference numerals have been used therefore for identical components, but with the addition of the letter "a". The apparatus according to Fig. 2 differs, however, from that described according to Fig. 1 in that the outlet side of the dialyzer 24a lacks a return conduit for the return of the whole blood to its source 25a. Instead the outlet side is connected to the conduit 33a after the measuring apparatus 35a via a conduit 38a for the collection of the whole blood in the fourth chamber 5 via the junction 20a on the junction block 23a, the conduit 21a and the connecting nipple 22a.

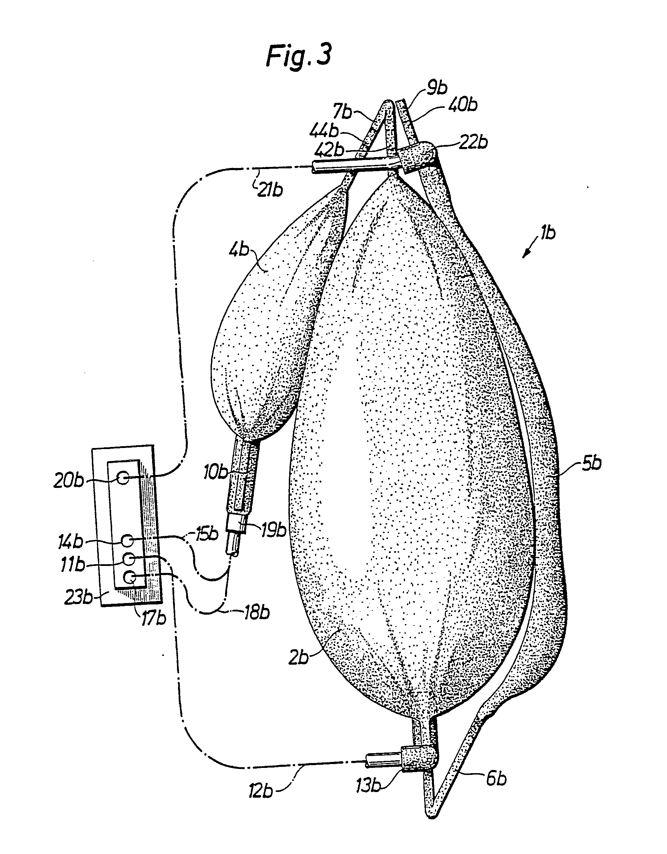

In Fig. 3 is shown a preferred embodiment of the container for the liquids forming part of the apparatus according to Fig. 1 and 2. The same reference numerals as in the two foregoing figures have therefore been used for corresponding components, but with the addition of the letter "b".

As can be seen from Fig. 3, the bag 1b is preferably folded along the welds 6b and 7b to a bellows-like packet with the chambers 2b, 3b (not shown), 4b and 5b arranged next to one another. The first chamber 2b for dialyzing solution is situated between on the one side the chambers 3b (not shown) for heparin and 4 for calibrating solution and on the other side the chamber 5b for the collection of spent liquid.

As shown in Fig. 1 (Fig. 2) holes 39, 40 (39a, 40a) and 41—44 (41a—44a) are provided in the welded zones 9 (9a) and 10 (10a) respectively.

If the container is folded in the manner as shown in Fig. 3, corresponding holes 39b, 40b and 41b—44b on the bag 1b will form two through-going holes, one of which, 40b, 42b, 44b, is indicated by broken lines at the top in Fig. 3. These two through-going holes 40b, 42b, 44b and 39b, 41b, 43b (not shown) respectively are intended to be used for suspending the bag on suspension hooks (not shown) or the like in the apparatus in accordance with the invention.

When the apparatus in accordance with the invention is used for measuring the concentration of glucose in whole blood, the procedure is as follows, with reference to Fig. 1.

In the first place the measuring electrode 35 is calibrated by allowing a calabrating solution of known content of the compound to be measured to flow from the chamber 4 of the bag 1 via connecting nipple 19, conduit 18, junction 17 on the junction block 23 and conduit 39, valve 34 and conduit 33 past the meauring electrode. Having passed the measuring electrode the calibrating solution is collected in the chamber 5 via the continuation of the conduit 33, junction 20 on the junction block 23, conduit 21 and the connecting nipple 22.

Whole blood is pumped from the source 25 with the help of pump 40 in conduit 26, 27 through the dialyzer 24 for the diffusion of glucose in the whole blood to the dialyzing solution which in a corresponding manner is pumped with the help of the pump 32 in the conduit 31 from the chamber 2 in the bag 1 via connecting nipple 13, conduit 12 and junction 11 in the junction block 23. To prevent coagulating of the whole blood, herapin is pumped at the same time with the help of a pump 41 in a conduit 42, forming part of the apparatus, connected to the junction 14 on the junction block 23 and the conduits 26, 27 at 43 from the chamber 3 of the bag 1 via the connecting nipple 16 on the conduit 15 connected to the junction 14 on the junction block 23. After passage through the dialyzer 24 the whole blood is returned to the source 25 through the conduit 28 via the air trap 29 and the clip 30. The part of the glucose which has diffused over into the dialyzing solution in the dialyzer 24 is conveyed with this solution out of the dialyzer through conduit 33, enzyme bed 37 via valve 34 and subsequently conduit 36 to the measuring apparatus 35 for measurement. After the measurement, spent dialyzing solution is conducted to the chamber 5 of the bag 1 in the same manner as described above in connection with the calibrating solution.

To obtain a reference valve for the measurement, the dialyzing solution is pumped at times from the dialyzer 24 directly to the measuring apparatus 35 via the valve 34 without passage through the enzyme bed 37. With the help of the value recorded by the measuring apparatus 35 a measure of the glucose concentration in the whole blood is obtained indirectly through proportionality, as the enzymatic conversion reaction involved in the enzyme bed 37 is known.

The apparatus in accordance with Fig. 2 in principle operates in the same manner as that in Fig. 1, the only difference being that the whole blood after passage through the dialyzer 24a is collected in the bag 1a instead of being returned to the source 25a. Thus the whole blood is conducted from the dialyzer 24a to the chamber 5a of the bag 1a via conduit 38a, conduit 33a, junction 20a on the junction block 23a, conduit 21a and connecting nipple 22a.

Industrial Applicability

The apparatus in accordance with the invention is usable for measuring the concentration of low-molecular compound in a complex medium through dialysis of the complex medium so as to form a dialyzate on which the measurement is carried out.

The apparatus can be used especially for measuring the concentration of glucose in whole blood, in particular in connection with medical treatment.

**Claims**

1. An apparatus for measuring the concentration of a low-molecular compound in a complex medium, particularly blood, this apparatus comprising a dialyzer (24; 24a; 24b) for the dialysis of the complex medium so as to form a dialyzate on which the measurement is to be carried out, a measuring device (35; 35a; 35b) for carrying out said measurement, and a common container (1; 1a; 1b) for the feed and removal, respectively, of dialyzing liquid and at least one further liquid useful for the measurement, wherein the container is divided into separate chambers (2, 3, 4, 5; 2a, 3a, 4a, 5a; 2b, 3b, 4b, 5b) for said liquids, characterized in that said container is folded along welded zones (6, 7; 6a, 7a; 6b, 7b) to a substantially bellows-like packet with the chambers arranged next to one another.

2. An apparatus in accordance with claim 1, characterized in that the container is made of a transparent material.

3. An apparatus in accordance with claim 1 or 2, characterized in that the welded zones (7, 9; 7a, 9a; 7b, 9b) comprise holes (39, 40, 41—44; 39a, 40a, 41a—44a; 39b, 40b, 41b—44b) which are arranged to form two through-going holes (40b, 42b, 44b; 41b, 43b) for the suspension of the container on corresponding suspension hooks.

**Revendications**

1. Appareil pour mesurer la concentration d'un composé de faible poids moléculaire dans un milieu complexe, en particulier du sang, l'appareil comprenant du dialyseur (24; 24a; 24b) pour la dialyse du milieu complexe, de façon à obtenir un dialysat sur lequel la mesure doit être effectuée, un dispositif de mesure (35; 35a; 35b) pour effectuer ladite mesure, et un récipient commun (1; 1a; 1b) pour l'alimentation et l'évacuation, respectivement, du liquide de dialyse et d'au moins et autre liquide utile pour la mesure, dans lequel ledit récipient est divisé en chambres séparées (2, 3, 4, 5; 2a, 3a, 4a, 5a; 2b, 3b, 4b, 5b) pour lesdits liquides, caractérisé en ce que le récipient est plié le long de zones soudées (6, 7; 6a, 7a; 6b, 7b) en un sac sensiblement en forme de soufflet, les chambres étant disposées les unes à la suite des autres.

2. Appareil suivant la revendication 1, caractérisé en ce que le récipient est fabriqué en une matière transparente.

3. Appareil suivant la revendication 1 ou 2, caractérisé en ce que les zones soudées (7, 9; 7a, 9a; 7b, 9b) comprennent des orifices (39, 40, 41—44; 39a, 40a, 41a—44a; 39b, 40b, 41b—44b) qui sont situés de manière à former deux trous traversants (40b, 42b, 44b; 41b, 43b) pour la suspension du récipient sur des crochets de suspension correspondants.

**Patentansprüche**

1. Vorrichtung zum Messen der Konzentration einer Verbindung mit niedrigem Molekulargewicht in einem komplexen Medium, insbesondere in Blut, wobei die Vorrichtung einen Dialysator (24; 24a; 24b) für die Dialyse des komplexen Mediums aufweist, um ein Dialysat zu bilden, an welchem die Messung ausgeführt werden soll, eine Meßvorrichtung (35; 35a; 35b) zur Ausführung der Messung und einen gemeinsamen Behälter (1; 1a; 1b) für die Zufuhr bzw. Entnahme von Dialysierflüssigkeit und mindestens einer weiteren Flüssigkeit, die für die Messung nützlich ist, wobei der Behälter in separate Kammern (2, 3, 4, 5; 2a, 3a, 4a, 5a; 2b, 3b, 4b, 5b) für die Flüssigkeiten geteilt ist, dadurch gekennzeichnet, daß der Behälter entlang geschweißten Zonen (6, 7; 6a, 7a; 6b, 7b) zu einem im wesentlichen balgartigen Paket gefaltet ist, wobei die Kammern nächst zueinander angeordnet sind.

2. Vorrichtung nach Anspruch 1, dadurch gekennzeichnet, daß der Behälter aus einem transparenten Material hergestellt ist.

3. Vorrichtung nach Anspruch 1 oder 2, dadurch gekennzeichnet, daß die geschweißten Zonen (7, 9; 7a, 9a; 7b, 9b) Löcher (39, 40, 41—44; 39a, 40a, 41a—44a; 39b, 40b, 41b—44b) aufweisen, die so angeordnet sind, daß sie zwei durchgehende Löcher (40b, 42b, 44b; 41b, 43b) für das Abhängen des Behälters auf entsprechenden Abhängehaken zu bilden.

Fig. 1

Fig. 2

# Fig.3